# FASCICULE DE BREVET EUROPEEN

(11) **EP 0 830 605 B1**
(45) Date de publication et mention de la délivrance du brevet: **30.08.2000**
(21) Numéro de dépôt: 96917552.0
(22) Date de dépôt: 24.05.1996
(51) Int. Cl.: G01N 33/576

(54) **METHODE DE PRONOSTIC DE L'EVOLUTION D'UNE INFECTION PAR LE VIRUS DE L'HEPATITE C, ET NECESSAIRE POUR SA MISE EN OEUVRE**
VERFAHREN ZUR VORHERSAGE DER ENTWICKLUNG EINER HEPATITIS-C-INFEKTION, UND TESTKIT ZUR DURCHFÜHRUNG DES VERFAHRENS
METHOD FOR THE PROGNOSIS OF THE EVOLUTION OF A HEPATITIS C VIRAL INFECTION AND KIT FOR IMPLEMENTING SAME

(30) Priorité: 26.05.1995 FR 9506248
(43) Date de publication de la demande: 25.03.1998
(73) Titulaire: PARTEUROP DEVELOPPEMENT, 69007 Lyon (FR); Negrier, Claude, 69680 Chassieu (FR); Tardy, Jean-Claude, 69600 Oullins (FR)
(72) Inventeur: NEGRIER, Claude, F-69680 Chassieu (FR); TARDY, Jean-Claude, F-69600 Oullins (FR)
(74) Mandataire: Tonnellier, Jean-Claude
(86) Numéro de dépôt international: FR9600788
(87) Numéro de publication internationale: WO9637783

(56) Documents cités:
- WO-A-91/15771
- WO-A-93/04089
- WO-A-94/25486

## Description

La présente invention a pour objet une méthode de pronostic du risque d'évolution vers un état chronique agressif, d'une infection par le virus de l'hépatite C, ainsi qu'un nécessaire pour la mise en oeuvre d'une telle méthode.

Les hépatites virales humaines peuvent avoir diverses origines. Les différents types de virus étaient répertoriés jusqu'en 1989 comme des virus de type A, de type B ou de type non A- non B.

Depuis 1989, deux virus particuliers ont été identifiés au sein du type non A-non B : le virus de type E, qui est responsable d'hépatites non A-non B à transmission essentiellement entérique et le virus de type C, qui est responsable d'hépatites non A-non B à transmission essentiellement parentérale. On considère généralement que 80 % des hépatites virales non A-non B sont des hépatites virales de type C.

Le virus de l'hépatite C (VHC) est un virus à ARN simple brin, de polarité positive, d'une taille de 9400 nucléotides. Le génome comporte quatre régions. La première région est une région dite non structurale répartie elle-même en plusieurs sous-régions correspondant à des gènes codant pour des protéines non structurales appelées NS1, NS2, NS3, NS4 et NS5. La deuxième région, dite structurale, est également divisée en différentes sous-régions correspondant à des gènes qui codent pour les protéines de capside et d'enveloppe. Enfin, les deux autres régions correspondent aux extrémités 5' et 3' non codantes et sont impliquées dans la régulation de la traduction du génome ; voir notamment à ce sujet A. Takamizawa et al., J. Virol., 65:1105-1113 (1991).

On estime généralement que 0,5 % à 1 % de la population des pays développés serait contaminé par le VHC.

On considère qu'un individu est infecté par le virus de l'hépatite C lorsque l'on peut détecter la présence d'un certain nombre de marqueurs sérologiques du VHC. On met généralement en évidence cette présence de marqueurs du VHC par un test de dépistage global d'anticorps dirigés contre un ensemble d'antigènes du VHC. Le test utilisé habituellement est un test du type ELISA, par exemple le test commercialisé sous la dénomination "Ortho HCV 3.0 ELISA TEST SYSTEM"® par la Société Ortho Diagnostic System.

On confirme généralement ce diagnostic par un second type de test dans lequel on recherche de façon séparée, dans des échantillons de sérum ou de plasma prélevés chez le sujet, des anticorps dirigés spécifiquement contre divers antigènes du VHC. Parmi les tests utilisés à cet effet, on peut citer notamment le test commercialisé sous la dénomination de "Chiron Riba HCV 3.0 SIA"® par la Société Ortho Diagnostic System. Ce test se présente sous forme de bandelettes sur lesquelles sont immobilisées, sous forme de bandes distinctes, différentes protéines permettant la recherche d'anticorps dirigés contre les protéines NS3, NS4, NS5 et une protéine de core qui est la protéine C-22. Ce test permet de confirmer l'infection au bout de trois mois environ après le contact infectant.

La notice de ce test indique simplement que la présence d'anticorps dirigés contre la protéine NS5 améliore le diagnostic des sujets infectés car elle constitue un marqueur supplémentaire.

La seule technique de diagnostic direct du VHC correspond à la mise en évidence de la présence de l'ARN viral. Néanmoins, l'ARN est un composé fragile car il est à la fois sensible à la température et susceptible d'être dégradé par les RNases qui sont omniprésentes, de sorte que sa recherche nécessite des conditions d'analyse très strictes. De telles contraintes limitent l'utilisation de cette technique.

Par ailleurs, on sait que pour environ 50 % des sujets infectés par le VHC, l'infection évolue vers un état chronique agressif pouvant conduire à une cirrhose, ou même à un carcinome hépato-cellulaire. On entend par "état chronique agressif' un état dans lequel on peut mettre en évidence des lésions histologiques du foie.

On considère actuellement qu'une infection par le VHC est au stade chronique agressif lorsque deux mesures réalisées à 6 mois d'intervalle montrent un taux de transaminases sériques égal ou supérieur à 1,5 fois le taux de transaminases sériques considéré comme normal. On entend par transaminases sériques, les transaminases glutamo-oxaloacétiques (S.G.O.T.) et les transaminases glutamo-pyruviques (S.G.P.T.). Parmi ces transaminases, on étudie en particulier l'alanine-aminotransférase (ALT) dont le taux sérique normal peut varier de 30 à 60 U.I./L, selon les modes opératoires propres à chaque laboratoire.

Actuellement, la caractéristique sérologique de l'état chronique agressif telle que définie ci-dessus constitue le critère requis pour la mise en oeuvre du principal traitement thérapeutique connu chez les sujets souffrant d'une hépatite C chronique, à savoir l'administration d'interféron qui permet d'obtenir une rémission durable chez les sujets répondeurs.

On sait que la mise en oeuvre précoce du traitement par l'interféron augmente les chances d'obtenir une meilleure réponse à ce traitement ; voir par exemple L. Pagliaro et al., Hepatology, Vol.16, 820-828 (1992).

Il serait donc particulièrement souhaitable de pouvoir disposer d'un marqueur prédictif d'une évolution de l'infection vers un état chronique agressif, avant même l'augmentation des transaminases sériques.

On a maintenant découvert que parmi les sujets déjà infectés et présentant un taux de transaminases normal, on pouvait distinguer deux populations significativement différentes, l'une possédant des anticorps dirigés contre la protéine NS5, l'autre n'en possédant pas.

On a en outre découvert en suivant ces deux populations que c'est uniquement parmi les sujets possédant des anticorps dirigés contre la protéine NS5 que l'on a observé ultérieurement une évolution vers l'état chronique agressif.

Les découvertes qui sont à la base de l'invention reposent sur les études décrites ci-après.

D'une part, on a étudié une population constituée par 83 sujets hémophiles infectés par le VHC et dont la contamination est estimée antérieure à 1987. En effet, le principal risque de contamination chez ces sujets était constitué par les nombreuses transfusions requises pour traiter leur hémophilie. Or, le dépistage du VHC est pratiqué en France depuis 1990, supprimant ou tout au moins réduisant très fortement, à partir de cette date, le risque de contamination par le VHC lors de transfusions. De plus, depuis 1987, le traitement viro-inactivant des produits sanguins (Facteur VIII, Facteur IX) a largement contribué à maîtriser les risques d'infection par le VHC.

Afin d'étudier cette population, on a effectué tous les 4 à 6 mois, pendant une période de 24 mois, les tests suivants :
(i) mesure du taux d'ALT,
(ii) test Chiron "Riba HCV 3.0 SIA"® tel que défini précédemment, et
(iii) recherche d'ARN viral par RT-PCR.

Parmi cette population, 41 sujets présentent un taux d'ALT considéré comme normal. Parmi ces sujets, 90,2 % possèdent des anticorps dirigés contre la protéine NS4, 97,5 % possèdent des anticorps dirigés contre la protéine NS3, 92,7 % possèdent des anticorps dirigés contre une protéine de core et 82,9 % possèdent de l'ARN viral détectable dans leur sérum tandis que seulement 43,9 % d'entre eux possèdent des anticorps dirigés contre la protéine NS5.

Par ailleurs, les 42 autres sujets étudiés possèdent un taux d'ALT élevé, supérieur ou égal à 65 U.I./L. Parmi ces sujets possédant un taux d'ALT élevé, 88,1 % possèdent des anticorps dirigés contre la protéine NS4, 100 % possèdent des anticorps dirigés contre la protéine NS3, 100 % possèdent des anticorps dirigés contre une protéine de core, 95,2 % possèdent de l'ARN viral détectable dans leur sérum et 83,3 % possèdent des anticorps dirigés contre la protéine NS5.

Ces résultats montrent donc que le sérum des sujets étudiés contient des anticorps dirigés contre les protéines NS3, NS4 et core, indépendamment de leur taux d'ALT. Par contre, la proportion de sujets possédant des anticorps dirigés contre la protéine NS5 dans leur sérum est statistiquement plus faible parmi la population présentant un taux d'ALT normal par rapport à la population présentant un taux d'ALT élevé.

D'autre part, le suivi de patients sur une période de deux ans a montré que ceux qui avaient à l'origine un taux d'ALT normal et dont l'infection a évolué, durant cette période, vers un état agressif chronique, provenaient tous d'un groupe de sujets qui, au début de l'étude, possédaient des anticorps dirigés contre la protéine NS5, alors que dans un groupe témoin constitué par des sujets infectés par le VHC mais qui présentaient un taux de transaminases normal et ne possédaient pas d'anticorps dirigés contre la protéine NS5, on n'a observé aucune augmentation du taux d'ALT, ni d'ailleurs aucune conversion vers une réactivité vis-à-vis de la protéine NS5, ni plus généralement aucune évolution vers un état chronique agressif.

Ces études montrent donc que la présence d'anticorps dirigés contre la protéine NS5 dans le sérum d'un sujet constitue un marqueur prédictif d'une évolution de l'infection par le VHC, chez ce sujet, vers un état chronique agressif. Ces études montrent en outre que la recherche de nombreux anticorps différents par des tests de type immunoblot comme le "Riba HCV 3.0 SIA" ne présente un intérêt, à titre de confirmation, que dans le cas d'un sujet consultant pour la première fois ou lorsque l'on soupçonne une primo-infection. Par contre, la recherche des anticorps dirigés contre les protéines NS3 et NS4 et contre les protéines de core, de même que la recherche de l'ARN viral, ne présentent aucun intérêt pour le suivi des patients infectés mais possédant un taux d'ALT normal. Pour ces sujets, seule la recherche des anticorps dirigés contre la protéine NS5 est intéressante, car elle permet, pour les patients donnant un résultat positif, d'envisager, à un stade précoce, le traitement à l'interféron (ou tout autre traitement antiviral et/ou immunostimulant approprié), compte tenu du fait que ce sont ces patients qui risquent d'évoluer vers un état chronique agressif.

La présente invention a donc pour objet une méthode de pronostic de l'évolution vers un état chronique agressif d'une infection par le virus de l'hépatite C chez un sujet déjà infecté présentant un taux de transaminases normal, cette méthode étant caractérisée par le fait que l'on recherche, de façon connue en soi, la présence d'anticorps dirigés contre la protéine NS5 du virus de l'hépatite C dans un échantillon biologique prélevé chez ledit sujet, et qu'en cas de présence desdits anticorps, on conclut à un risque d'évolution vers un état chronique.

Dans le cas où la recherche des anticorps anti-NS5 est négative, on peut en principe conclure à l'absence d'un tel risque.

La recherche d'anticorps particuliers dans un échantillon, par exemple un échantillon sérique, peut être réalisée de différentes façons selon des techniques bien connues.

Selon un mode de réalisation particulier, la méthode de pronostic telle que définie précédemment peut comprendre les étapes consistant à :
(i) faire incuber un échantillon biologique prélevé chez ledit sujet, dans des conditions permettant la formation de complexes du type antigène-anticorps, en présence d'au moins un réactif capable d'être reconnu spécifiquement par des anticorps dirigés contre la protéine NS5, et
(ii) mettre en évidence la présence de tels complexes antigène-anticorps.

Le réactif utilisé est notamment un polypeptide, par exemple la protéine NS5 elle-même. Cette protéine est connue et sa séquence a été décrite par exemple par P. Simmonds et al., J. Gen. Virol. 75, 1053-1061 (1994).

Selon un autre mode de réalisation, le polypeptide utilisé comprend au moins un peptide capable d'être reconnu spécifiquement par des anticorps dirigés contre la protéine NS5. Il s'agit par exemple d'un peptide comprenant une séquence peptidique incluse dans celle de la protéine NS5. Les peptides qui conviennent peuvent être facilement déterminés selon des techniques de routine bien connues.

Généralement, le polypeptide utilisé est constitué par la protéine NS5 ou par un ou plusieurs fragments de ladite protéine. Mais il peut également comprendre une séquence peptidique supplémentaire. Il peut par exemple comprendre un fragment d'une superoxyde dismutase ou tout autre fragment protéique jugé intéressant pour la mise en oeuvre de la méthode selon l'invention. Ces peptides peuvent être préparés par des techniques bien connues, notamment par l'intermédiaire d'ADN recombiné.

Pour la recherche des anticorps dirigés contre la protéine NS5, le polypeptide peut être immobilisé sur un support solide par tout moyen connu pour l'immobilisation des protéines, par exemple par adsorption, par affinité immuno-chimique (soit directement, soit indirectement par liaison avec une molécule intermédiaire) ou par liaison chimique, en particulier par liaison covalente soit directement, soit indirectement (par exemple par l'intermédiaire d'un agent de couplage bifonctionnel), etc.

Le terme "support solide" tel qu'utilisé ici inclut tous les matériaux classiques sur lesquels peut être immobilisé un réactif tel qu'un polypeptide. Ces matériaux sont connus et décrits dans la littérature. Parmi les matériaux solides capables de fixer les peptides par adsorption, on peut citer par exemple le polystyrène, le polypropylène, les latex, etc.

Parmi les matériaux utilisables pour fixer les peptides par covalence à l'aide d'un agent de couplage, on peut citer notamment le dextran, la cellulose ou leurs dérivés aminés tels que la diéthylaminoéthylcellulose ou le diéthylaminoéthyl-dextran.

Les agents de couplage permettant de fixer par covalence les peptides sur le support solide sont connus ; ce sont par exemple des dérivés carboxyliques, des dérivés bifonctionnels tels que les dialdéhydes par exemple le glutaraldéhyde, des diisocyanates par exemple le toluène diisocyanate, des quinones par exemple la benzoquinone, etc.

Le support peut se présenter par exemple sous forme de disque, de tube, de baguette, de bille, de feuille, de cône ou de plaque.

Parmi les méthodes pouvant être utilisées pour mettre en évidence la présence d'anticorps dirigés contre la protéine NS5 chez un sujet, on peut notamment utiliser une méthode dite indirecte. Ainsi, on fait incuber un échantillon biologique (notamment sérum sanguin) provenant du sujet dont on souhaite prédire si l'infection par le VHC va évoluer vers un état chronique, en présence d'un polypeptide tel que défini précédemment, immobilisé sur un support solide, puis on lave pour éliminer tous les composés de l'échantillon qui n'ont pas une affinité spécifique pour ledit polypeptide. On peut détecter la présence d'anticorps dirigés contre la protéine NS5 fixés, selon les méthodes connues, par exemple à l'aide d'anticorps marqués dirigés contre un déterminant antigénique isotypique des immunoglobulines humaines, et on examine si les anticorps marqués sont fixés ou non sur le support (étape de révélation). Si l'anticorps marqué se trouve fixé sur le support, c'est que sur le support s'est formé le complexe polypeptide-anticorps dirigés contre la protéine NS5 et que, par conséquent, l'anticorps dirigé contre la protéine NS5 recherché était présent dans l'échantillon examiné. On peut donc en déduire que le sujet chez qui on a prélevé l'échantillon présente un risque significatif de développer une forme chronique d'hépatite C. Dans le cas contraire, c'est-à-dire lorsque l'anticorps couplé au marqueur ne s'est pas fixé sur le support, on peut conclure à l'absence d'anticorps dirigés contre la protéine NS5 dans l'échantillon testé et donc que le sujet ne présente pas, au moment du test, de risque d'évolution vers une hépatite C chronique.

On peut également mettre en évidence la présence d'anticorps dirigés contre la protéine NS5 dans le sérum du sujet par une méthode dite de "compétition".

Pour cela, on peut par exemple mettre en contact le polypeptide tel que défini précédemment, immobilisé sur un support solide, avec un échantillon de sérum à tester. Après lavage pour éliminer les produits non fixés, on met en contact le polypeptide avec une quantité définie d'anticorps marqués dirigés contre la protéine NS5 couplés à un marqueur. On lave de nouveau, puis on examine le système pour rechercher la présence ou l'absence de l'anticorps marqué. Si l'anticorps marqué s'est fixé sur le système, c'est que les sites du polypeptide capables d'être reconnus par des anticorps dirigés contre la protéine NS5 étaient libres et que par conséquent, le sérum à tester ne contenait pas d'anticorps dirigés contre la protéine NS5 capables de réagir avec le polypeptide fixé au support. Si l'anticorps marqué ne se fixe pas ou ne se fixe qu'en faible proportion, par exemple si la quantité fixée est inférieure de plus de 50 % à la quantité fixée dans le cas d'un sérum témoin ne contenant pas l'anticorps recherché, c'est que le sérum étudié contenait le même anticorps que celui couplé au marqueur, et que les complexes anticorps-antigène formés ont masqué les sites capables d'être reconnus par l'anticorps dirigés contre la protéine NS5 et ont donc empêché la fixation de l'anticorps marqué sur le polypeptide fixé au support.

On peut également mettre en évidence la présence d'anticorps dirigés contre la protéine NS5 dans le sérum du sujet par une méthode dite de "sandwich vrai".

Pour cela, on peut par exemple mettre en contact le polypeptide tel que défini précédemment, immobilisé sur un support solide, avec un échantillon de sérum à tester. Après incubation et lavage pour éliminer les produits non fixés, on ajoute le polypeptide NS5 couplé à un marqueur. Après incubation et lavage, on examine le système pour rechercher la présence ou l'absence d'antigène (polypeptide NS5) marqué. Si le polypeptide NS5 marqué est fixé sur le système ainsi obtenu, cela prouve la présence d'anticorps anti-NS5 dans l'échantillon de sérum à tester. Inversement, l'absence de fixation, sur ce système, du polypeptide NS5 marqué, prouve l'absence d'anticorps anti-NS5 dans l'échantillon de sérum à tester.

Les incubations décrites précédemment pour les différentes méthodes sont effectuées en laissant incuber pendant un temps suffisant, par exemple 1 heure, à température convenable, comprise par exemple entre 37 et 40°C environ.

Le marqueur utilisé dans la méthode selon l'invention peut être par exemple un marqueur radioactif, fluorescent, enzymatique, etc.

Le couplage des anticorps avec un marqueur est connu en soi. Dans le cas d'un marquage enzymatique, l'enzyme est par exemple une peroxydase ou une phosphatase alcaline. L'activité enzymatique éventuellement présente après réalisation du test tel que décrit précédemment peut être déterminée selon les méthodes connues avec un substrat approprié (agent révélateur) permettant par exemple une révélation par colorimétrie, par fluorescence, par luminescence, etc.

La présente invention a également pour objet un nécessaire pour la mise en oeuvre de la méthode de pronostic telle que définie précédemment, comprenant un réactif capable d'être reconnu par des anticorps dirigés contre une protéine du virus de l'hépatite C, ledit nécessaire comprenant un seul tel réactif, et ledit réactif étant capable d'être reconnu spécifiquement par des anticorps dirigés contre la protéine NS5, et non par des anticorps dirigés contre d'autres protéines du virus de l'hépatite C.

En d'autres termes, ce test ne contient, comme antigènes susceptibles d'être reconnus par des anticorps du VHC, que des antigènes susceptibles d'être reconnus par des anticorps dirigés contre la protéine NS5.

Le réactif tel que défini précédemment peut être constitué d'un seul type de polypeptide capable d'être reconnu spécifiquement par des anticorps dirigés contre la protéine NS5, ou d'un mélange de tels polypeptides.

Bien entendu, le nécessaire selon l'invention peut comprendre d'autres réactifs polypeptidiques tels que notamment des anticorps anti-immunoglobulines humaines, servant par exemple à révéler la présence d'immunoglobulines humaines fixées sur un support solide lors de la réalisation d'un test de pronostic conformément à l'invention.

Le nécessaire selon l'invention peut également comprendre un support solide tel que défini précédemment.

Le réactif capable d'être reconnu spécifiquement par des anticorps dirigés contre la protéine NS5 est par exemple immobilisé ou immobilisable sur le support solide. L'immobilisation dudit réactif peut être réalisé par tout moyen connu d'immobilisation de protéines, comme décrit précédemment.

Selon un mode de réalisation particulier du nécessaire selon l'invention, ledit nécessaire peut contenir en outre les autres réactifs et ingrédients permettant la mise en oeuvre de la méthode de pronostic telle que définie précédemment selon la technique ELISA. La technique ELISA (Enzyme-Linked Immunoadsorbent Assay) et ces autres réactifs et ingrédients nécessaires à sa mise en oeuvre sont connus. Ce sont notamment des anticorps anti-(immuno-globuline humaine) marqués avec une enzyme, un agent révélateur, des solutions de lavage, etc.

L'invention concerne également l'utilisation d'un test de recherche des anticorps anti-NS5 dans une méthode de prise de décision du traitement d'un patient infecté par le VHC. Le traitement peut comprendre tout traitement antiviral et/ou immunostimulant approprié connu. La décision de traitement est prise lorsque le test, effectué sur un sujet infecté ayant un taux de transaminases normal, par exemple un taux d'ALT normal, montre la présence d'anticorps anti-NS5.

## Revendications

1. Méthode de pronostic de l'évolution vers un état chronique agressif d'une infection par le virus de l'hépatite C chez un sujet déjà infecté présentant un taux de transaminases normal, caractérisée par le fait que l'on recherche, de façon connue en soi, la présence d'anticorps dirigés contre la protéine NS5 du virus de l'hépatite C dans un échantillon biologique prélevé chez ledit individu et qu'en cas de présence desdits anticorps, on conclut à un risque d'évolution vers un état chronique agressif.

2. Méthode selon la revendication précédente, caractérisée par le fait qu'elle comprend les étapes consistant à :
(i) faire incuber un échantillon biologique prélevé chez ledit sujet, dans des conditions permettant la formation de complexes du type antigène-anticorps, en présence d'au moins un réactif capable d'être reconnu spécifiquement par des anticorps dirigés contre la protéine NS5, et
(ii) mettre en évidence la présence de tels complexes antigène-anticorps.

3. Méthode selon la revendication 2, caractérisée par le fait que ledit réactif est un polypeptide.

4. Méthode selon la revendication 3, caractérisé par le fait que ledit polypeptide est la protéine NS5 ou comprend au moins un peptide ayant une séquence peptidique incluse dans celle de ladite protéine.

5. Nécessaire pour la mise en oeuvre de la méthode de pronostic telle que définie dans l'une des revendications 1 à 4, comprenant un réactif capable d'être reconnu par des anticorps dirigés contre une protéine du virus de l'hépatite C, caractérisé par le fait qu'il comprend un seul tel réactif, et que ledit réactif est capable d'être reconnu spécifiquement par des anticorps dirigés contre la protéine NS5, et non par des anticorps dirigés contre d'autres protéines du virus de l'hépatite C.

6. Nécessaire selon la revendication 5, caractérisé par le fait que ledit réactif est tel que défini dans l'une quelconque des revendications 3 et 4.

7. Nécessaire selon la revendication 5 ou 6, caractérisé par le fait que ledit réactif est immobilisé ou immobilisable sur un support solide.

8. Nécessaire selon la revendication 7, caractérisé par le fait qu'il contient en outre des réactifs et ingrédients permettant la mise en oeuvre de ladite méthode selon la technique ELISA.

## Patentansprüche

1. Prognoseverfahren über die Entwicklung eines chronisch aggressiven Zustands einer Hepatitis C-Virusinfektion in einem damit infizierten Subjekt, das einen normalen Transaminasegrad aufweist, dadurch gekennzeichnet, dass man auf an sich bekannte Weise die Anwesenheit von gegen das NS5 Protein des Hepatitis C-Virus gerichtete Antikörper in einer dem Individuum entnommenen biologischen Probe untersucht und für den Fall der Anwesenheit dieser Antikörper auf das Risiko der Entwicklung eines chronisch aggressiven Zustands schließt.

2. Verfahren gemäß dem vorstehenden Anspruch, dadurch gekennzeichnet, dass es die Schritte umfasst, bestehend aus:
(i) Inkubieren-lassen einer biologischen Probe, die dem Subjekt entnommen wurde, unter Bedingungen, welche die Bildung eines Komplexes vom Typ Antigen-Antikörper gestatten, in Anwesenheit mindestens eines Reagens, das spezifisch gegen das NS5 Protein gerichtete Antikörper erkennen kann, und
(ii) Nachweis der Anwesenheit dieser Antigen/Antikörper-Komplexe.

3. Verfahren gemäß Anspruch 2, dadurch gekennzeichnet, dass das Reagens ein Polypeptid ist.

4. Verfahren gemäß Anspruch 3, dadurch gekennzeichnet, dass das Polypeptid das NS5 Protein darstellt oder mindestens ein Peptid mit einer Peptidsequenz umfasst, die in dem Protein enthalten ist.

5. Kit zur Durchführung des Prognoseverfahrens, wie es in einem der Ansprüche 1 bis 4 definiert ist, umfassend ein Reagens, das gegen ein Protein des Hepatitis C-Virus gerichtete Antikörper erkennen kann, dadurch gekennzeichnet, dass er nur ein solches Reagens enthält und dass dieses Reagens spezifisch gegen das NS5 Protein gerichtete Antikörper erkennen kann und nicht gegen andere Proteine des Hepatitis C-Virus gerichtete Antikörper.

6. Kit gemäß Anspruch 5, dadurch gekennzeichnet, dass das Reagens gemäß einem der Ansprüche 3 und 4 definiert ist.

7. Kit gemäß Anspruch 5 oder 6, dadurch gekennzeichnet, dass das Reagens auf einem festen Träger immobilisiert oder immobilisierbar ist.

8. Kit gemäß Anspruch 7, dadurch gekennzeichnet, dass er zusätzlich Reagenzien und Bestandteile umfasst, die die Durchführung des Verfahrens gemäß der ELISA-Technik gestatten.

## Claims

1. Method of prognosticating that an infection with the hepatitis C virus, in a subject who is already infected and who is exhibiting a normal level of transaminase, is progressing towards an aggressive chronic state, characterized in that the presence of antibodies directed against the NS5 protein of the hepatitis C virus is sought, in a manner known per se, in a biological sample taken from the said individual, and in that, if the said antibodies are present, it is concluded that there is a risk of progression towards an aggressive chronic state.

2. Method according to the preceding claim, characterized in that it comprises the seeps consisting of:
(i) incubating a biological sample taken from the said subject under conditions which permit the formation of complexes of the antigen/antibody type in the presence of at least one reagent which is capable of being recognized specifically by antibodies directed against the NS5 protein, and
(ii) demonstrating the presence of such antigen/antibody complexes.

3. Method according to Claim 2, characterized in that the said reagent is a polypeptide.

4. Method according to Claim 3, characterized in that the said polypeptide is the NS5 protein or comprises at least one peptide which has a peptide sequence which is included in that of the said protein.

5. Kit for implementing the prognostication method as defined in one of Claims 1 to 4 and comprising a reagent which is capable of being recognized by antibodies directed against a protein of the hepatitis C virus, characterized in thac it comprises only one such reagent, and in that the said reagent is capable of being recognized specifically by antibodies directed against the NS5 protein and not by antibodies directed against other proteins of the hepatitis C virus.

6. Kit according to Claim 5, characterized in that the said reagent is as defined in either of Claims 3 and 4.

7. Kit according to Claim 5 or 6, characterized in that the said reagent is immobilized or immobilizable on a solid support.

8. Kit according to Claim 7, characterized in that it furthermore contains reagents and ingredients which enable the said method to be implemented in accordance with the ELISA technique.
